# EUROPEAN PATENT SPECIFICATION

(11) **EP 2 213 651 B1**
(45) Date of publication and mention of the grant of the patent: **19.12.2012**
(21) Application number: 08800667.1
(22) Date of filing: 10.10.2008
(51) Int. Cl.: C07C 213/10, C07C 217/74, C07B 57/00

(54) **RESOLUTION OF 4,5-DIMETHOXY-1-(METHYLAMINOMETHYL)-BENZOCYCLOBUTANE**
OPTISCHE TRENNUNG VON 4,5-DIMETHOXY-1-(METHYLAMINOMETHYL)BENZOCYCLOBUTAN
RÉSOLUTION DE 4,5-DIMÉTHOXY-1-(MÉTHYLAMINOMÉTHYL)-BENZOCYCLOBUTANE

(30) Priority: 16.11.2007 CN 200710188301; 19.05.2008 CN 200810093266
(43) Date of publication of application: 04.08.2010
(73) Proprietor: Jiangsu Hengrui Medicine Co., Ltd., Jiangsu 222002 (CN)
(72) Inventor: SUN, Piaoyang, Jiangsu 222002 (CN); CHEN, Yongjiang, Jiangsu 222002 (CN); YU, Guangliang, Jiangsu 222002 (CN)
(74) Representative: Dilg, Haeusler, Schindelmann Patentanwaltsgesellschaft mbH
(86) International application number: PCT/CN2008/001711
(87) International publication number: WO 2009/062377

(56) References cited:
- EP-A1- 1 598 333
- CN-A- 1 699 331
- US-A- 5 296 482
- SIMON, H. ET AL.: "O,O'-Di-p-Toluoyl-(2R,3R)-Tartaric Acid as Supramolecular Resolving Agent", JOURNAL OF THERMAL ANALYSIS AND CALORIMETRY, vol. 74, no. 1, 2003, pages 155-162, XP019254761, ISSN: 1572-8943
- FERRAYOLI C, C.G. ET AL.: "RESOLUTION OF RACEMIC ALBUTEROL VIA DIASTEREOMERIC SALTS FORMATION WITH DI-P-TOLUOYL-D-TARTARIC ACID", ENANTIOMER, vol. 5, no. 3-04, 2000, pages 289-291, XP008050542, ISSN: 1024-2430
- EVANS G R ET AL: "Highly efficient resolution of (+/-)-Tramadol with di-p-toluoyl-tartaric acid (DTTA)", TETRAHEDRON ASYMMETRY, PERGAMON PRESS LTD, OXFORD, GB, vol. 12, no. 11, 4 July 2001 (2001-07-04), pages 1663-1670, XP027377826, ISSN: 0957-4166 [retrieved on 2001-07-04]

## Description

### FIELD OF THE INVENTION

The present invention provides a resolving method of 4,5-dimethoxy-1-(methylaminomethyl)-benzocyclobutane (II) as an important intermediate of Ivabradine to obtain an intermediate of Ivabradine i.e., (1S)-4,5-dimethoxy-1-(methylaminomethyl)-benzocyclobutane (I).

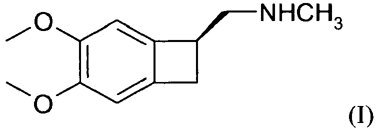

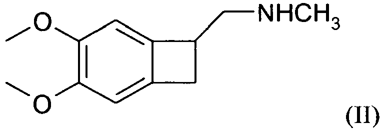

A compound of formula (I), which was prepared according to the method of the present invention, can be used for the synthesis of Ivabradine.

### BACKGROUND OF THE INVENTION

Ivabradine and pharmaceutical acceptable salts thereof, particularly hydrochloride thereof, have very valuable pharmacological and therapeutic effects, especially a bradycardiac effect, so that these compounds can be used not only in the treatment or prevention of various clinical diseases of myocardial ischemia such as angina, myocardial infarction and associated rhythm disorders, but also for the treatment or prevention of various rhythm disorders, especially supraventricular rhythm disorder. Ivabradine is an effective drug of treatment of myocardial ischemia such as angina pectoris and so on.

United States Patent US 5,296,482 describes a synthetic route of Ivabradine in detail. Furthermore, the patent describes a resolving method of compounds of formula (II) to obtain a compound of formula (I), using camphor sulfonic acid. But the result of the resolving method is far from satisfactory. Firstly, the resolving method has a very low yield by using camphor sulfonic acid as a resolving agent, and the yield is only 4-5%, which results in the total yield of only 2-3%. Such a low yield leads to high cost and low efficiency. Secondly, because of the low selectivity of resolving agent, the enantiomer excess (ee) value of resolving product is low and the product needs several re-crystallizing steps to complete the resolution; furthermore, the process is complicated and not suitable for the industrial production, and the chiral purity of final product is not satisfactory either.

European patent application EP 1 598 333 A1 describes a further method for the synthesis of (1S)-4,5-dimethoxy-1-(methylaminomethyl)-benzocyclobutane (ivabradine), which employs optically active diacidic aminoacid compounds as resolving agents, in particular N-acetyl-L-glutamic acid. The cumulative overall yield obtained by this method is 30%.

In view of the medicinal value of Ivabradine and salts thereof, it's very necessary to find an effective industrial method to obtain intermediate compound of formula (I) of S configuration with high chiral purity in high efficiency and high yield.

### DETAILED DESCRIPTION OF THE INVENTION

After extensive study, the inventor has found that various acidic resolving agents commonly used are substantially invalid for the resolution of racemate of formula (II), except that R-camphor sulfonic acid has certain selectivity. Some can not react with the racemate of formula (II) to get crystallization and precipitate in various solvents effectively, such as L-tartaric acid, R-mandelic acid, and so on; some can react with the racemate of formula (II) to get crystallization and precipitation in a solvent, but there is no selectivity and the resulting precipitation are still mixture, such as N-acetyl-L-glutamic acid, L-leucine, and so on.

After extensive study, the inventor has surprisingly found that in a large number of conventional acidic resolving agents tested, only tartaric acid diacylated by benzoyl or substituted benzoyl, such as dibenzoyl-L-tartaric acid (L-DBTA), dibenzoyl-D-tartaric acid (D-DBTA), di-*p*-toluoyl-L-tartaric acid (L-DTTA) or di-*p*-toluoyl-D-tartaric acid (D-DTTA), can separate (S)-amine and (R)-amine effectively.

The present invention relates to a resolving method of an important intermediate of Ivabradine i.e., 4,5-dimethoxy-1-(methyl-amino-methyl)-benzocyclobutane (II) (intermediate amine, a racemate), comprising the step of reacting a resolving agent with said intermediate to get corresponding salt in alcohol solvents or alcohol aqueous solution, followed by the crystallization step, wherein said resolving agent is dibenzoyl-L-tartaric acid (L-DBTA), dibenzoyl-D-tartaric acid (D-DBTA), di-*p*-toluoyl-L-tartaric acid (L-DTTA) or di-*p*-toluoyl-D-tartaric acid (D-DTTA), and preferably di-*p*-toluoyl-L-tartaric acid, or di-*p*-toluoyl-D-tartaric acid. Finally, the aimed product (1S)-4,5-dimethyloxy-1-(methylaminomethyl)-benzocyclobutane of formula (I) is obtained.

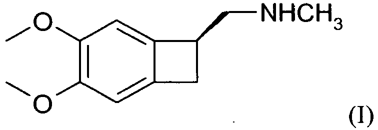

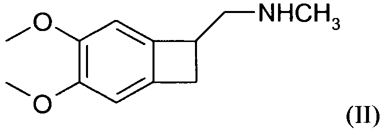

Further, the resolving method of the present invention includes re-crystallizing step after salifying and crystallizing steps. The resolving agent, di-*p*-toluoyl-L-tartaric acid (L-DTTA) and di-*p*-toluoyl-D-tartaric acid (D-DTTA), used in the present invention may be used alone or jointly. Specifically, the present invention relates to a resolving method of racemate of formula (II). The problem to be solved by the present invention is to obtain the above optically pure pharmaceutical acceptable compound of formula (I) of S configuration with excellent yield by using di-*p*-toluoyl-L-tartaric acid. The method is characterized in that the racemate of formula (II) react with an acidic resolving agent in a particular solvent to get corresponding salt and to precipitate a desired crystal of chiral intermediate amine salt selectively.

The resolving method of compounds of formula (II), not only refers to compounds of formula (II) reacting with an acidic chiral resolving agent to get corresponding salt, but also comprises the steps of crystallization, re-crystallization, extraction to obtain intermediate amine of formula (I), if necessary, further comprises the step of compound of formula (I) reacting with appropriate acid to get the corresponding salt as a suitable intermediate for synthesis of Ivabradine. Compounds of formula (II) used herein are prepared according to the U.S. Patent US 5,296,482.

With regard to the amount of the resolving agent, in theory, according to the correspondence of acid-base reaction, the molar ratio of the intermediate amine to the resolving agent can be 2:1; if only considering the desired configuration, the molar ratio can be 4:1; if considering acid addition salts prepared in molar equivalence, the molar ratio can be 1:1. However, after research, the inventor found that a higher proportion of the resolving agent has more satisfactory yield to obtain a resolving product with high chiral purity. Generally speaking, the suitable molar ratio of the intermediate amine to the resolving agent can be from 5:1 to 1:2, the preferable ratio is from 2:1 to 1:1, and the excessive resolving agent is not more helpful for the resolution.

Said resolving process of racemate of formula (II) is carried out in an alcohol solvent. Moreover, the alcohol solvent could be used alone or combined with other organic solvents together. Alcohols solvents, used in the present invention, include alcohol used alone as well as alcohol-based solvent mixtures. Said alcohol solvents are lower fatty alcohol of less than 3 carbon atoms, preferably ethanol. Said alcohol solution is ethanol aqueous solution. Further, the resolving process of racemate of formula (II) can also be carried out in an alcohol aqueous solution. The proportion between alcohol and water can be arbitrary; preferably the proportion of alcohol solvents is 50% ∼ 100%. Alcohol solvents here are lower fatty alcohol of less than 3 carbon atoms, preferably ethanol.

In order to improve the chiral purity of intermediate amine of formula (I), sometimes it is necessary for the compound of formula (I) to be re-crystallized. The resolving process can generally be carried out at room temperature; if necessary, under heat condition. Generally, the re-crystallizing step is carried out under heat condition, firstly the compound of formula (I) is dissolved in a particular solvent, and then the re-crystalization is completed slowly at room temperature. In general, after re-crystallizing twice, the chiral purity is often satisfactory, and the ee value is generally above 99%.

The process to get free intermediate amine is conventional, wherein alkali used preferably is sodium hydroxide; wherein the extraction solvent used is a hydrophobic organic solvent used in conventional extraction, preferably toluene, ethyl acetate, methylene chloride and chloroform etc., more preferably ethyl acetate and chloroform. Moreover, the process of salification of compound of formula (I) is also conventional, wherein the salt may be formed with a pharmaceutical acceptable acid and the compound of formula (I) in order to purify, store and directly react, and the acid used in salification is preferably hydrochloric acid. The salifying method is conventional, and it is easy for the person professionally trained in the art.

The compound of formula (I) or salts thereof according to the present invention has the cumulative resolving yield up to more than 80% and the chiral purity more than 99%, particularly suitable as a synthetic intermediate of Ivabradine and pharmaceutical acceptable salts thereof.

### PREFERRED EMBODIMENTS

The present invention is illustrated by the following examples in detail

### EXAMPLE 1

8.0 g (41.40 mmol) of racemate of formula (II) was dissolved in 350 ml of ethanol. To this reaction mixture, 4.0 g (10.35 mmol) of L-DTTA was added. Then, the reaction mixture was heated to reflux until the initially un-dissolved material thereby went into the solution. After cooling, the solid was crystallized from the reaction solution. The resulting precipitate was collected by filtration, and then was dried to obtain a crude product. It was analyzed by HPLC, showing 94.14% of S configuration.

The crude product was dissolved in 350 ml of ethanol. The reaction mixture was heated to reflux until the initially un-dissolved material thereby went into the solution. After cooling, the solid was crystallized from the reaction solution. The resulting precipitate was collected by filtration, and then was dried to obtain 3.47 g (5.99 mmol) of product with the resolving yield of 28.9% (the proportion of S configuration in relation to the racemate by weight). It was analyzed by HPLC, showing 97.19% of S configuration.

### EXAMPLE 2

8.0 g (41.40 mmol) of racemate of formula (II) was dissolved in 350 ml of ethanol. To this reaction mixture, 8.0 g (20.7 mmol) of L-DTTA was added. Then, the reaction mixture was heated to reflux until the initially un-dissolved material thereby went into the solution. After cooling, the solid was crystallized from the reaction solution. The resulting precipitate was collected by filtration, and then was dried to obtain a crude product. It was analyzed by HPLC, showing 96.84% of S configuration.

The crude product was dissolved in 350 ml of ethanol. The reaction mixture was heated to reflux until the initially un-dissolved material thereby went into the solution. After cooling, the solid was crystallized from the reaction solution. The resulting precipitate was collected by filtration, and then was dried to obtain 4.3 g (7.42 mmol) of product with the resolving yield of 35.8%. It was analyzed by HPLC, showing 99.87% of S configuration.

### EXAMPLE 3

8.0 g (41.40 mmol) of racemate of formula (II) was dissolved in 350 ml of ethanol. To this reaction mixture, 16.0 g (41.4 mmol) of L-DTTA was added. Then, the reaction mixture was heated to reflux until the initially un-dissolved material thereby went into the solution. After cooling, the solid was crystallized from the reaction solution. The resulting precipitate was collected by filtration, and then was dried to obtain a crude product. It was analyzed by HPLC, showing 94.56% of S configuration.

The crude product was dissolved in 350 ml of ethanol. The reaction mixture was heated to reflux until the initially un-dissolved material thereby went into the solution. After cooling, the solid was crystallized from the reaction solution. The resulting precipitate was collected by filtration, and then was dried to obtain 7.4 g (12.77 mmol) of first resolving product with the resolving yield of 61.7%. It was analyzed by HPLC, showing 99.25% of S configuration.

The resulting mother liquor during the above resolving process and re-crystallization was combined, and then concentrated to dryness to obtain a solid. Firstly, the solid was dealt with sodium hydroxide. Secondly, the reaction mixture was extracted with ethyl acetate to obtain 4.7 g of racemate of formula (II) mainly with R configuration. It was analyzed by HPLC, showing 73.5% of R configuration. Then it was dissolved in 350 ml of ethanol, and 14.9 g D-DTTA was added for the inverse resolution. The reaction mixture was heated to reflux until the initially un-dissolved material thereby went into the solution. After cooling, the solid was crystallized from the reaction solution. The resulting precipitate was collected by filtration, and then was dried to obtain a crude product. It was analyzed by HPLC, showing 94.86% of R configuration.

The crude product of R configuration was dissolved in 350 ml of ethanol. The reaction mixture was heated to reflux until the initially un-dissolved material thereby went into the solution. After cooling, the solid was crystallized from the reaction solution. The resulting precipitate was collected by filtration, and then was dried to obtain 7.3 g of product of R configuration with the reversed resolving yield of 60.8%. It was analyzed by HPLC, showing 99.38% of R configuration.

The resulting mother liquor during the above resolving process and re-crystallization was combined, and then concentrated to dryness to get a solid. Firstly, the solid was dealt with sodium hydroxide. Secondly, the reaction mixture was extracted with ethyl acetate to obtain 2.6 g of racemate of formula (II) mainly with S configuration. It was analyzed by HPLC, showing 55.5% of S configuration. Then it was dissolved in 100 ml of ethanol, and 5.0 g of L-DTTA was added. The reaction mixture was heated to reflux until the initially un-dissolved material thereby went into the solution. After cooling, the solid was crystallized from the reaction solution. The resulting precipitate was collected by filtration, and then was dried to obtain a crude product. It was analyzed by HPLC, showing 92.62% of S configuration.

The crude product was dissolved in 100 ml of ethanol. The reaction mixture was heated to reflux until the initially un-dissolved material thereby went into the solution. After cooling, the solid was crystallized from the reaction solution. The resulting precipitate was collected by filtration, and then was dried to obtain 2.5 g (4.31 mmol) of product. It was analyzed by HPLC, showing 99.10% of S configuration.

The product obtained after twice normal resolution was combined, and then it was dealt with sodium hydroxide aqueous solution. The reaction mixture was extracted with ethyl acetate to obtain 3.3 g of intermediate amine of formula (I) of S configuration with the overall resolving yield 82.5%. It was analyzed by HPLC, showing 99.20% of S configuration.

### EXAMPLE 4

8.0 g (41.40 mmol) of racemate of formula (II) was dissolved in 350 ml of ethanol. To this reaction mixture, 7.4 g (20.7 mmol) of L-DBTA was added. Then, the reaction mixture was heated to reflux until the initially un-dissolved material thereby went into the solution. After cooling, the solid was crystallized from the reaction solution. The resulting precipitate was collected by filtration, and then was dried to obtain a crude product. It was analyzed by HPLC, showing 83.37% of R configuration.

The crude product was dissolved in 350 ml of ethanol. The reaction mixture was heated to reflux until the initially un-dissolved material thereby went into the solution. After cooling, the solid was crystallized from the reaction solution. The resulting precipitate was collected by filtration, and then was dried to obtain 3.6 g of product. It was analyzed by HPLC, showing 90.78% of R configuration.

## Claims

1. A resolving method of an intermediate of Ivabradine, 4,5-dimethoxy-1-(methylaminomethyl)-benzocyclobutane (II), comprising the steps of reacting a resolving agent with said intermediate to obtain a corresponding salt in alcohol solvents or alcohol aqueous solution, followed by a crystallization step, wherein the resolving agent is a tartaric acid diacylated by benzoyl or substituted benzoyl, and wherein the target product obtained is (1S)-4,5-dimethyl-oxy-1-(methyl-amino-methyl)-benzo cyclobutane having formula (I):

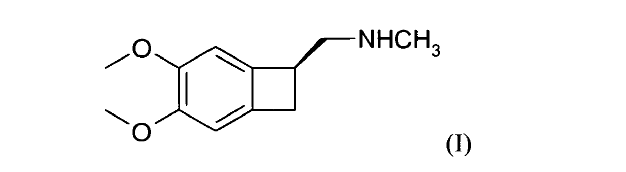

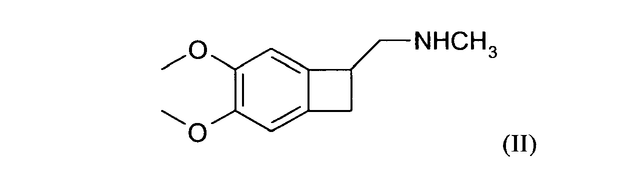

2. The resolving method according to claim 1, wherein the resolving agent is selected from the group consisting of: dibenzoyl-L-tartaric acid, dibenzoyl-D-tartaric acid, di-*p*-toluoyl-L-tartaric acid, and di-*p*-toluoyl-D-tartaric acid.

3. The resolving method according to claim 2, wherein the resolving agent is selected from the group consisting of: di-*p*-toluoyl-L-tartaric acid (L-DTTA), and di-*p*-toluoyl-D-tartaric acid (D-DTTA).

4. The resolving method according to any one of claims 1 to 3, wherein the resolving method further includes a re-crystallization step after the crystallization step.

5. The resolving method according to claim 3 or 4, wherein di-*p*-toluoyl-L-tartaric acid (L-DTTA) or di-*p*-toluoyl-D-tartaric acid (D-DTTA) as the resolving agent is used alone.

6. The resolving method according to claim 3 or 4, wherein di-*p*-toluoyl-L-tartaric acid (L-DTTA) or di-*p*-toluoyl-D-tartaric acid (D-DTTA) as the resolving agent are used jointly.

7. The resolving method according to any one of claims 1 to 6, wherein the alcohol solvents include alcohol used alone or alcohol-based solvent mixtures.

8. The resolving method according to any one of claims 1 to 7, wherein the alcohol solvent is ethanol, and the alcohol aqueous solution is an ethanol aqueous solution.

## Patentansprüche

1. Verfahren zur optischen Trennung eines Intermediats von Ivabradin, 4,5-Dimethoxy-1-(methylaminomethyl)-benzocyclobutan (II), welches den Schritt des Umsetzens eines optischen Trennmittels mit dem Intermediat umfasst, um in alkoholischen Lösungsmitteln oder einer wässrigen alkoholischen Lösung ein entsprechendes Salz zu erhalten, gefolgt von einem Kristallisierungsschritt, wobei das optische Trennmittel eine mit Benzoyl oder substituiertem Benzoyl diacylierte Weinsäure ist, und wobei das erhaltene Endprodukt (1S)-4,5-dimethyl-oxy-1-(methylaminomethyl)-benzocyclobutan mit der Formel (I) ist:

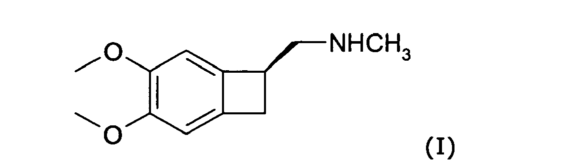

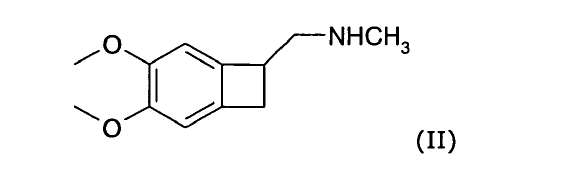

2. Verfahren zur optischen Trennung gemäß Anspruch 1, wobei das optische Trennmittel aus der Gruppe ausgewählt wird, die aus Dibenzoyl-L-weinsäure, Dibenzoyl-D-weinsäure, Di-*p*-toluoyl-L-weinsäure und Di-p-toluoyl-D-weinsäure besteht.

3. Verfahren zur optischen Trennung gemäß Anspruch 2, wobei das optische Trennmittel aus der Gruppe ausgewählt wird, die aus Di-*p*-toluoyl-L-weinsäure (L-DTTA) und Di-*p*-toluoyl-D-weinsäure (D-DTTA) besteht.

4. Verfahren zur optischen Trennung gemäß einem der Ansprüche 1 bis 3, wobei das optische Trennverfahren ferner nach dem Kristallisierungsschritt einen Re-kristallisierungsschritt umfasst.

5. Verfahren zur optischen Trennung gemäß Anspruch 3 oder 4, wobei Di-p-toluoyl-L-weinsäure (L-DTTA) oder Di-*p*-toluoyl-D-weinsäure (D-DTTA) als alleiniges optisches Trennmittel verwendet wird.

6. Verfahren zur optischen Trennung gemäß Anspruch 3 oder 4, wobei Di-p-toluoyl-L-weinsäure (L-DTTA) und Di-*p*-toluoyl-D-weinsäure (D-DTTA) gemeinsam als optisches Trennmittel verwendet werden.

7. Verfahren zur optischen Trennung gemäß einem der Ansprüche 1 bis 6, wobei die alkoholischen Lösungsmittel Alkohol, der alleine verwendet wird, und alkohol-basierte Lösungsmittelgemische umfassen.

8. Verfahren zur optischen Trennung gemäß einem der Ansprüche 1 bis 7, wobei das alkoholische Lösungsmittel Ethanol ist und die wässrige alkoholische Lösung eine wässrige Ethanol-Lösung ist.

## Revendications

1. Procédé de résolution d'un intermédiaire de l'Ivabradine, le 4,5-diméthoxy-1-(méthylaminométhyl)-benzocyclobutane (II), comprenant les étapes consistant à faire réagir un agent de résolution avec ledit intermédiaire pour obtenir un sel correspondant dans des solvants alcooliques ou de la solution aqueuse d'alcool, suivie d'une étape de cristallisation, dans lequel l'agent de dédoublement est un acide tartrique diacylé par un groupe benzoyle ou benzoyle substitué, et dans lequel le produit est obtenu cible (1S)-4,5-diméthyl-oxy-1-(méthylaminométhyl) - benzocyclobutane formule générale (I):

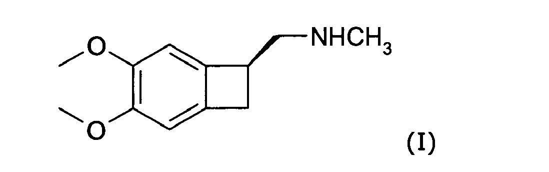

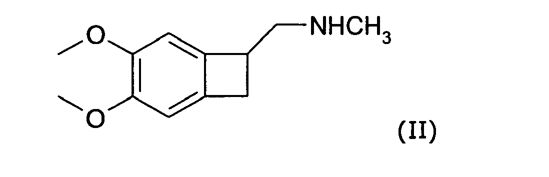

2. Procédé la résolution selon de la revendication 1, dans lequel l'agent de résolution est choisi dans le groupe constitué par: l'acide dibenzoyl-L-tartrique, l'acide dibenzoyl-D-tartrique, l'acide di-p-toluoyl-L-tartrique, et le di-p- toluoyl-D-tartrique.

3. Procédé la résolution selon de la revendication 2, dans lequel l'agent de résolution est choisi dans le groupe constitué par: le di-p-toluoyl-L-tartrique (L-DTTA), et le di-p-toluoyl-D-tartrique (D- DTTA).

4. Procédé la résolution selon de l'une quelconque des revendications 1 à 3, dans lequel le procédé comprend en outre la résolution d'une étape de recristallisation après l'étape de cristallisation.

5. Procédé la résolution selon de la revendication 3 ou 4, dans lequel le di-p-toluoyl-L-tartrique (L-DTTA) ou di-p-toluoyl-D-tartrique (D-DTTA) comme agent de résolution est utilisé seul.

6. Procédé la résolution selon de la revendication 3 ou 4, dans lequel le di-p-toluoyl-L-tartrique (L-DTTA) ou di-p-toluoyl-D-tartrique (D-DTTA) comme agent de résolution sont utilisés conjointement.

7. Procédé la résolution selon de l'une quelconque des revendications 1 à 6, dans lequel les solvants de type alcool comprennent l'alcool utilisé seul ou un alcool à base de mélanges de solvants.

8. Procédé la résolution selon de l'une quelconque des revendications 1 à 7, dans lequel le solvant alcool est l'éthanol, l'alcool et la solution aqueuse est une solution aqueuse d'éthanol.
